Europäisches Patentamt

European Patent Office

Office européen des brevets

(19)

(11) Veröffentlichungsnummer: **0 274 649 B1**

(12) **EUROPÄISCHE PATENTSCHRIFT**

(45) Veröffentlichungstag der Patentschrift: 24.04.91

(51) Int. Cl.⁵: **D06H 3/00**, D06C 9/00, D06C 13/00

(21) Anmeldenummer: 87117877.8

(22) Anmeldetag: 03.12.87

(54) **Verfahren zum Ausmessen der geometrischen Oberflächenstruktur von Flächengut und Vorrichtung zur Durchführung des Verfahrens.**

(30) Priorität: 18.12.86 DE 3643297
20.01.87 DE 3701394

(43) Veröffentlichungstag der Anmeldung:
20.07.88 Patentblatt 88/29

(45) Bekanntmachung des Hinweises auf die
Patenterteilung:
24.04.91 Patentblatt 91/17

(84) Benannte Vertragsstaaten:
BE CH DE ES GB IT LI

(56) Entgegenhaltungen:
FR-A- 1 502 035
GB-A- 791 477
US-A- 3 218 844

(73) Patentinhaber: **Osthoff Senge GmbH & Co. KG**
**Essener Strasse 62**
**W-5600 Wuppertal(DE)**

(72) Erfinder: **Ebbinghaus, Rainer**
**Grunewaldstrasse 84**
**W-4050 Mönchengladbach(DE)**
Erfinder: **Hohenester, Otmar**
**Birkenhöhe 13**
**W-5600 Wuppertal 1(DE)**

(74) Vertreter: **Peerbooms, Rudolf, Dipl.-Phys.**
**Postfach 200 208 Dickmannstrasse 45c**
**W-5600 Wuppertal 2(DE)**

## Beschreibung

Die Erfindung betrifft ein Verfahren zum Ausmessen der geometrischen Oberflächenstruktur von Flächengut, insbesondere im Zusammenhang mit der Überwachung der verbleibenden Haarigkeit beim Absengen oder Scheren von textilem Flächengut, sowie eine Vorrichtung zur Durchführung des Verfahrens. (siehe FR-A- 1 502 035)

Zur Verbesserung der Trage- und Gebrauchseigenschaften von Textilien ist es bekannt, das textile Flächengut, speziell Gewebebahnen, auf einer Sengmaschine zu behandeln, wobei abstehende und nicht im Faden eingebundene Faserenden abgebrannt werden. Hierdurch wird eine saubere Oberfläche erzielt, die weniger leicht anschmutzt und weniger zur Pillingbildung neigt. Das Absengen wird mit einer sehr heißen Flamme durchgeführt, wobei die Flammentemperatur und die Vorbeilaufgeschwindigkeit des textilen Flächengutes derart aufeinander abgestimmt sein müssen, daß einerseits die freien Faserenden möglichst vollständg abgebrannt werden, andrerseits aber das Grundgewebe nicht thermisch beansprucht wird. Die bekannten Sengmaschinen lassen sich zwar auf die für ein bestimmtes textiles Flächengut geeigneten Konditionen einregulieren, jedoch wäre es wünschenswert, den Grad der Faserabsengung laufend automatisch überwachen zu können, um so den Sengprozeß auf Einhaltung eines konstanten Sengergebnisses regeln zu können.

Ähnliche Überwachungsprobleme treten auch bei vielen anderen Arten von Flächengut auf, das mit einer bestimmten Oberflächenstruktur versehen werden soll, beispielsweise beim Beschichten von Papierbahnen oder bei der Herstellung von Tuftingbahnen. In solchen Fällen wird meist die Einhaltung einer bestimmten Schichtdichte bzw. Florhöhe verlangt.

Der Erfindung liegt die Aufgabe zugrunde, ein Verfahren zum Ausmessen der geometrischen Oberflächenstruktur von Flächengut anzugeben, mit dem sehr genaue Werte erhalten werden und das mit wirtschaftlich vertretbaren Kosten realisierbar ist.

Zur Lösung dieser Aufgabe ist erfindungsgemäß vorgesehen, daß das Flächengut auf einer Umlenkfläche gebogen und an seiner zu überwachenden Oberflächenseite gespreizt wird und daß seine Oberfläche an mindestens einer Stelle mit einem tangential die Oberfläche streifenden Lichtstrahl abgetastet und nach der Dunkelfeldmethode mittels eines fotoelektrischen Sensors höhenmäßig ausgemessen wird. Dieses Verfahren ermöglicht es, beispielsweise eine Gewebebahn nach dem Sengen kontinuierlich längs einer Meßspur auf die verbliebene Haarigkeit zu überwachen, wobei jedes an der Meßstelle auftretende Haar erfaßt und in seiner Höhe ausgemessen werden kann. Die elektirsche Auswertschaltung wird dabei so ausgelegt, daß die Oberseite des Grundgewebes als Bezugspunkt "Null" gewählt ist, so daß die darüber hinaus ragende Höhe der Fasern exakt ausgemessen und beispielsweise auf einem Bildschirm kontinuierlich angezeigt werden kann.

In der Praxis reicht es insbesondere bei den modernen Hochleistungs-Gassengmaschinen aus, die Haarigkeit nur längs einer Spur zu überwachen. Der Erfindung zufolge kann die Oberfläche aber auch an mehreren Stellen mittels mehrerer paralleler Lichtstrahlen abgetastet werden, welche entweder auf denselben fotoelektrischen Sensor hingelenkt werden oder die jeweils auf einen gesonderten fotoelektrischen Sensor gerichtet sind.

Gemäß einer alternativen Ausführungsart des Meßverfahrens kann der Erfindung zufolge vorgesehen werden, daß der Lichtstrahl parallel zur Biegeachse der Umlenkfläche gerichtet wird. Durch diese Maßnahme ist es nunmehr möglich, mit einem einzigen Lichtstrahl über die gesamte Flächengutbreite hinweg die mittlere Höhe beispielsweise der vorstehenden Haare zu ermitteln, wobei aber nunmehr auch alle über die Flächengutbreite und Länge verteilte, die verlangte Sollhöhe übersteigende Haare oder dgl. erfaßt werden. Aus der Häufigkeit, mit der über die Sollhöhe vorstehende Haare oder dgl. erfaßt werden, können Rückschlüsse auf die Qualität des Flächengutes geschlossen und im Rahmen einer Prozeßregelung entsprechende Korrektureingriffe ausgelöst werden.

Bei der Überwachung der Haarigkeit oder Faserhöhe von textilem Gut kann in Weiterbildung der Erfindung vorgesehen werden, daß das textile Gut im Bereich der Meßstelle einem elektrostatischen, seine Fasern aufrichtenden Feld ausgesetzt wird. Durch diese Maßnahme wird sichergestellt, daß alle freien Faserenden bei der Ausmessung erfaßt werden und daß darauf aufmerksam gemacht wird, wenn bei speziellen Gewebearten der Sengvorgang nicht effektiv ausgeführt wird und beispielsweise noch Vorbehandlungen an der Gewebebahn zur Vorbereitung auf den Sengvorgang ausgeführt werden müssen.

Die nach dem Verfahren der Erfindung gewonnenen Meßwerte für die Faserhöhe können zur Regelung von Gassengmaschinen ausgenutzt werden, indem die Laufgeschwindigkeit der Gewebebahn und/oder die Sengflammentemperatur auf Einhaltung einer konstanten Faserhöhe nachgeregelt werden. Bei Gewebeschermaschinen kann die Regelung der Faserhöhe durch entsprechendes Verstellen des Scherzylinders erfolgen.

Zur Durchführung des Verfahrens nach der Erfindung wird eine Vorrichtung vorgesehen, die eine rotierende oder starre Meßtrommel aufweist, über die das Flächengut geführt wird, und die ferner eine längs einer Trommeltangente angeordnete optische Sensoreinrichtung aufweist, welche aus einer auf einer Trommelseite angeordneten Lichtquelle und aus einer auf der anderen Trommelseite angeordneten Diodenzeile besteht, de senkrecht zur Tangentialebene gerichtet ist. Als besonders geeignet haben sich hierbei CCD (charge coupled devices) -Diodenzeilen erwiesen, deren lichtempfindliche Fläche ein Rechteck von beispielsweise nur einigen Zehnteln Millimetern Breite und einigen Millimetern Höhe ist, wobei über die Höhe hinweg mehrere hundert diskrete Sensorpunkte bei modernen Diodenzeilen vorgesehen sind, so daß eine sehr feine Auflösung erreicht wird. Anstelle einer Diodenzeile kann der Erfindung zufolge auch ein Diodenarray verwendet werden, was bei der Überprüfung körniger Oberflächenstrukturen vornehmlich in Frage kommt.

In weiterer Ausgestaltung der Erfindung kann vorgesehen werden, daß die Sensoreinrichtung längs der Trommel hin und her verfahrbar ist, so daß also das Flächengut nicht nur immer längs einer Meßspur, sondern auch auf voller Breite überwacht werden kann. Statt einer verfahrbaren Sensoreinrichtung kann auch vorgesehen werden, daß an einer Trommelseite mehrere Lichtquellen angeordnet sind, denen auf der anderen Trommelseite entweder Spiegel zur Umlenkung der Lichtstrahlen auf dieselbe Diodenzeile oder jeweils individuelle Diodenzeilen zugeordnet sind.

Zur Durchführung der alternativen Ausführungsart des Verfahrens sieht die Erfindung eine Vorrichtung vor, bei der die Umlenkfläche von einem feststehenden Schwert oder von einer umlaufenden Meßtrommel gebildet ist und bei der an einem Längsende des Schwertes bzw. der Meßtrommel ein optischer Sensor, z.B. ein Laserstrahler, und am anderen Ende ein optischer Empfänger angeordnet sind, wobei der Sender und der Empfänger zur Anpassung an die Dicke bzw. Sollhöhe der Warenbahn synchron höhenverstellbar sind.

Die Erfindung wird im folgenden anhand einer Zeichnung näher erläutert. In der Zeichnung zeigt :
Fig. 1 eine schematische Darstellung einer Vorrichtung zum Ausmessen der geometrischen Oberflächenstruktur von Flächengut,
Fig. 2 eine mit der Vorrichtung nach Fig. 1 gewonnene Meßkurve für eine gesengte Gewebebahn,
Fig. 3 eine Meßkurve für eine ungesengte Gewebebahn,
Fig. 4 eine schematische Darstellung einer zweiten Ausführungsform einer Vorrichtung zum Ausmessen der geometrischen Oberflächenstruktur von Flächengut, wobei das Flächengut über eine Trommel geführt wird, und
Fig. 5 ein drittes Ausführungsbeispiel, bei dem das Flächengut über ein feststehendes Umlenkschwert geführt wird.

Das hinsichtlich seiner Oberflächenstruktur auszumessende Flächengut, beispielsweise eine gesengte Gewebebahn 1, wird über eine Umlenkfläche 2 geführt, wobei es sich beim gezeigten Ausführungsbeispiel um eine angetriebene Meßtrommel 3 handelt. Auf der Umlenkfläche 2 erfährt die Gewebebahn 1 an ihrer zu beobachtenden Oberseite eine Spreizung, so daß dort die zu erfassenden, abstehenden Fasern 4 durch einen tangential gerichteten Lichtstrahl 5 abgetastet werden können. Von einer Lichtquelle 6 wird das Licht durch eine, mehrere Linsen 7, 8, 9, die Blende 10 und einen Tubus 11 mit Blende umfassende, optische Einrichtung auf eine Zeilendiode 12 gerichtet, an welcher jeweils die im Zenit der Trommel 3 befindlichen Fasern abgebildet werden.

Je nach Höhe der vom Lichtstrahl abgetasteten Faser werden mehr oder weniger Einzeldioden der Diodenzeile belichtet bzw. abgedunkelt, und die Auswertschaltung 13 liefert an ihren Ausgang A eine Spannung, die proportional zur Faserlänge ist.

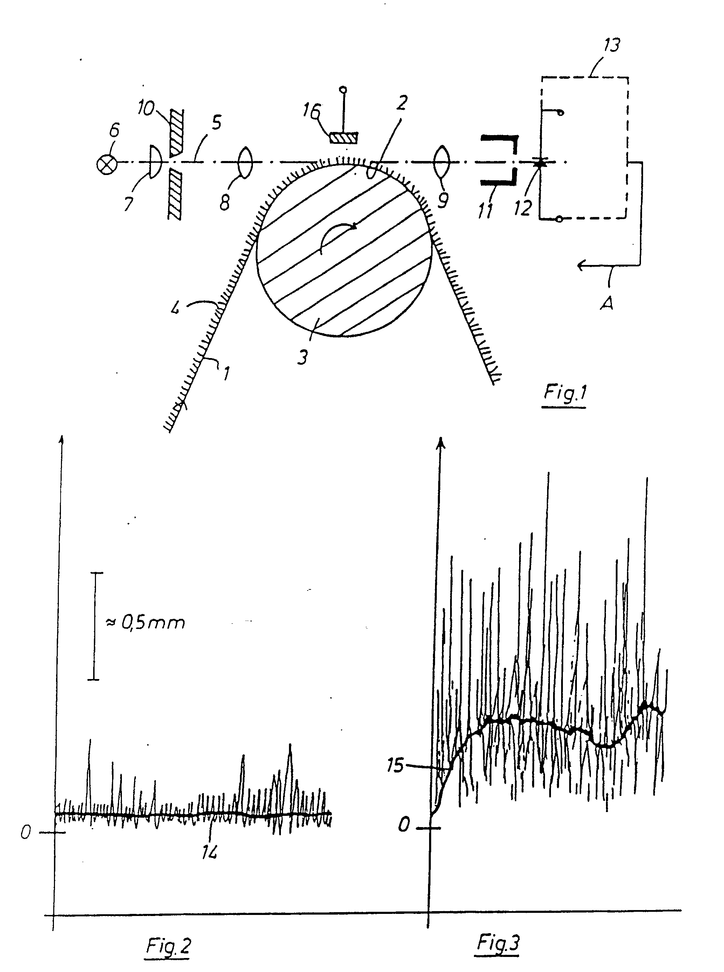

Der Ausgang der Auswertschaltung 13 kann aufgezeichnet werden, und Fig. 2 zeigt die längs einer Meßspur erfaßten Fasern bei einer gesengten Gewebebahn. In Fig. 3 ist zum Vergleich die Faserhöhe und Faserverteilung gezeigt, die die Gewebebahn vor dem Sengen aufwies. Die dicken Linien 14, 15 geben jeweils die Mittelwerte der Faserhöhe in den Fig. 2 und 3 an. Der Ausgang A der Auswertschaltung 13 kann zu Regelungszwecken oder auch nur zur Auslösung eines Warnsignales oder dgl. verwendet werden.

Wie in Fig. 1 noch angedeutet ist, befindet sich oberhalb der Meßstelle an der Trommel 3 eine Elektrode 16, mit der ein elektrostatisches Feld erzeugt wird, das auf eine Aufrichtung der Fasern 4 hinwirkf.

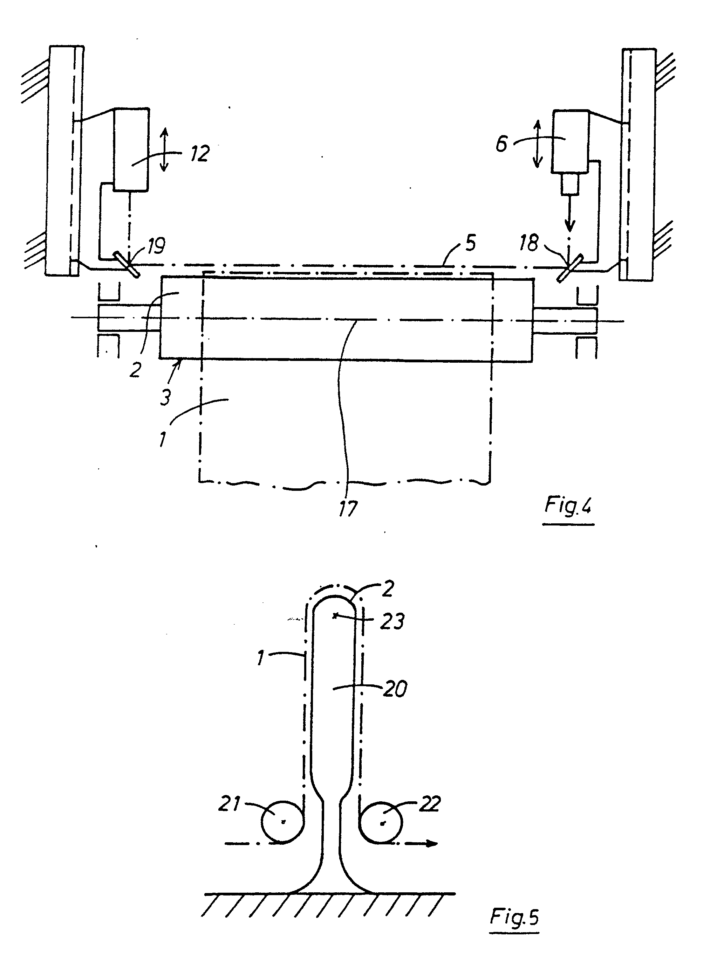

Beim Ausführungsbeispiel nach Fig. 4 wird das auszumessende Flächengut, beispielsweise eine gesengte Gewebebahn 1, über eine Umlenkfläche 2 geführt, wobei es sich wiederum um eine angetriebene Meßtrommel 3 handelt. Auf der Umlenkfläche erfährt die Gewebebahn 1 an ihrer zu beobachtenden Oberseite eine Spreizung, so daß dort die zu erfassenden, abstehenden Fasern durch einen tangential gerichteten Lichtstrahl 5 abgetastet werden können. Der Lichtstrahl 5 ist hier parallel zur Achse 17 der Meßtrommel gerichtet. Von einer Lichtquelle 6, z.B. einem Laserstrahlsender, wird der Lichtstrahl dabei über die Spiegel 18, 19 umgelenkt und auf den Empfänger, z.B. eine Zeilendiode 12, gerichtet. Der Sender 6 und der Empfänger 12 sind an den beiden Längsenden der Meßtrommel 3 angeordnet und synchron höhenverstellbar, um eine Einstellung entsprechend der Dicke der Gewebebahn 1 vornehmen zu können. Beim Ausführungsbeispiel nach Fig. 1 ist eine Meßwalze 3 mit hoher Rundlaufgenauigkeit vorzusehen.

Alternativ kann die Umlenkfläche 2 auch von einem feststehenden Umlenkschwert 20 gebildet sein, über dessen oberen Scheitel die Gewebebahn 1 mittels Umlenkrollen 21, 22 gelenkt wird. Mit Position 23 ist die Krümmungsachse für den oberen Scheitelbereich des Schwertes 20 angegeben. Beim Ausführungsbeispiel nach Fig. 5 ist der Lichtstrahl 5 parallel zu dieser Achse 23, d. h. also senkrecht zur Darstellungsebene nach Fig. 1 gerichtet.

**Ansprüche**

1. Verfahren zum Ausmessen der geometrischen Oberflächenstruktur von Flächengut, insbesondere zur Überwachung der verbleibenden Haarigkeit beim Absengen oder Scheren von textilem Flächengut, dadurch gekennzeichnet, daß das Flächengut (1) auf einer Umlenkfläche (2) gebogen und an seiner zu überwachenden Oberflächenseite gespreizt wird und daß seine Oberfläche an mindestens einer Stelle mit einem tangential die Oberfläche streifenden Lichtstrahl (5) abgetastet und nach der Dunkelfeldmethode mittels eines fotoelektrischen Sensors (12) höhenmäßig ausgemessen wird.

2. Verfahren nach Anspruch 1, dadurch gekennzeichnet, daß das Flächengut (1) kontinuierlich über eine Meßtrommel (3) geführt wird und daß der Lichtstrahl (5) parallel zu den Diametralebenen der Meßtrommel (3) gerichtet ist.

3. Verfahren nach den Ansprüchen 1 und 2, dadurch gekennzeichnet, daß die Oberflächen mit mehreren parallelen Lichtstrahlen abgetastet wird, welche entweder auf denselben fotoelektrischen Sensor hingelenkt oder jeweils auf einen gesonderten fotoelektrischen Sensor gerichtet sind.

4. Verfahren nach Anspruch 1, dadurch gekennzeichnet, daß der Lichtstrahl (5) parallel zur Krümmungsachse (17, 23) der Umlenkfläche (2) gerichtet ist.

5. Verfahren nach einem der Ansprüche 1, 2, 3 oder 4, bei Anwendung für textiles Gut, dadurch gekennzeichnet, daß das textile Flächengut im Bereich der Meßstelle einem elektrostatischen, seine Fasern aufrichtenden Feld ausgesetzt wird.

6. Verfahren nach einem oder mehreren der Ansprüche 1 bis 5, dadurch gekennzeichnet, daß die Faserhöhe bei Sengmaschinen durch Anpassen der Laufgeschwindigkeit der Gewebebahn und/oder der Sengflammentemperatur und bei Gewebeschermaschinen durch Verstellen des Scherzylinders jeweils in Abhängigkeit von einer Abweichung zwischen der gemessenen Ist-Faserhöhe und der Soll-Faserhöhe geregelt wird.

7. Vorrichtung zur Durchführung des Verfahrens nach Anspruch 1, gekennzeichnet durch eine das Flächengut tragende Meßtrommel(3), über die das Flächengut (1) geführt wird, und durch eine längs einer Trommeltangente angeordneten optischen Sensoreinrichtung (6 bis 12), welche eine auf einer Trommelseite angeordnete Lichtquelle (6) und eine auf der anderen Trommelseite angeordneten Diodenzeile (12) umfaßt, die senkrecht zur Tangentialebene gerichtet ist.

8. Vorrichtung nach Anspruch 7, dadurch gekennzeichnet, daß die Sensoreinrichtung (6 bis 12) längs der Trommel (3) hin und her verfahrbar ist.

9. Vorrichtung nach Anspruch 7, dadurch gekennzeichnet, daß an einer Trommelseite mehrere Lichtquellen (6) angeordnet sind, denen auf der anderen Trommelseite entweder Spiegel zur Umlenkung der Lichtstrahlen auf dieselbe Diodenzeile (12) oder jeweils individuelle Diodenzeilen zugeordneten sind.

10. Vorrichtung zur Durchführung des Verfahrens nach Anspruch 1, dadurch gekennzeichnet, daß die Umlenkfläche (2) von einem feststehenden Schwert (20) oder von einer umlaufenden Meßtrommel (3) gebildet ist und daß an einem Längsende des Schwertes (20) bzw. der Meßtrommel (3) eine Lichtquelle (6), z.B. ein Laserstrahler, und am anderen Ende ein optischer Empfänger (12), z.B. eine Zeilendiode, angeordnet sind, wobei der Sender und der Empfänger synchron höhenverstellbar sind.

**Claims**

1. Method for measuring the geometric properties of a web, more particularly for monitoring residual hairiness when singeing or cropping textile web, characterised in that the web (1) is bent around a direction-changing surface (2) and, on the side to be monitored, is spread, and in that its surface is scanned at least at one point by a beam of light (5) striking the surface at a tangent and its height measured, using the dark field method, by means of a photo-electric sensor (12).

2. Method according to Claim 1, characterised in that the web (1) is continuously passed over a measurement drum (3) and in that the beam of light (5) is directed parallel to the diametric planes of the measuring drum (3).

3. Method according to Claims 1 and 2, characterised in that the surfaces are scanned by several parallel beams of light, which are either linked to the same photo-electric sensor or are each directed towards a separate photo-electric sensor.

4. Method according to Claim 1, characterised in that the beam of light (5) is directed parallel to the bend axis (17,23) of the direction-changing surface (2).

5. Method according to one of Claims 1,2,3 or 4 when used for textile web, characterised in that the textile web is exposed in the area of the measuring point to an electrostatic field which sets its fibres on end.

6. Method according to one or more of Claims 1 to 5, characterised in that the height of the fibres is regulated when using singeing machines by adjusting the running speed of the roll of fabric and/or the temperature of the singeing flames and when using cropping machines by adjusting the cropping cylinder, in each case depending on the discrepancy between the actual measured height of the fibres and the desired height of the fibres.

7. Apparatus for implementing the method according to Claim 1, characterised by a measuring drum (3) carrying the web, over which the web (1) is fed, and by a sensor arrangement (6 to 12) along a tangent to the drum, which includes a light source (6) arranged on one side of the drum and a row of diodes (12) arranged on the other side of the drum perpendicular to the tangential plane.

8. Apparatus according to Claim 7, characterised in that the sensor arrangement (6 to 12) is moveable backwards and forwards along the drum (3).

9. Apparatus according to Claim 7, characterised in that on one side of the drum several light sources (6) are arranged, to which on the other side of the drum there are either corresponding mirrors for diverting the light beams onto the same row of diodes (12) or onto individual rows of diodes.

10. Apparatus for implementing the method according to Claim 1, characterised in that the direction-changing surface (2) takes the form of an immobile tongue (20) or of a rotating measurement drum (3) and in that at one end of the tongue (20) or of the measurement drum (3) a light source (6), for example a laser, and at the other end an optical receiver (12), for example a damping diode, are arranged, the height of transmitter and receiver being synchronously adjustable.

**Revendications**

1. Procédé de mesure de la structure géométrique d'une surface de produit plat, en particulier pour le contrôle de l'abondance de poils restant lors du flambage ou de la tonte de produit textile plat, caractérisé par le fait qu'on cintre le produit plat (1) sur une surface déflectrice (2) et on l'écarte sur son côté de surface à contrôler et qu'on palpe sa surface, en au moins un emplacement, avec un rayon lumineux (5) rasant tangentiellement la surface et on la mesure en hauteur au moyen d'un capteur photoélectrique (12) selon la méthode sur fond noir.

2. Procédé selon la revendication 1, caractérisé par le fait qu'on fait passer le produit plat (1) en continu sur un tambour de mesure (3) et on dirige le rayon lumineux (5) parallèlement au plan diamétral du tambour de mesure (3).

3. Procédé selon les revendications 1 et 2, caractérisé par le fait qu'on palpe la surface avec plusieurs rayons lumineux parallèles qu'on dirige tous sur le même capteur photoélectrique ou qu'on dirige respectivement chacun sur un capteur photoélectrique particulier.

4. Procédé selon la revendication 1, caractérisé par le fait que le rayon lumineux (5) est orienté parallèlement à l'axe de courbure (17, 23) de la surface déflectrice (2).

5. Procédé selon l'une des revendications 1, 2, 3 ou 4, pour application à un produit textile, caractérisé par le fait qu'on soumet le produit textile plat, dans la zone de l'emplacement de mesure, à un champ électrostatique redressant ses fibres.

6. Procédé selon l'une ou plusieurs des revendications 1 à 5, caractérisé par le fait que l'on règle la hauteur de fibre, pour les machines de flambage, en ajustant la vitesse de marche de la bande de tissu et/ou de la température de flamme de flambage et, pour les machines à tondre, en réglant le cylindre de tonte, dans chaque cas en fonction d'un écart entre la hauteur de fibres réelle mesurée et la hauteur de fibres de consigne.

7.  Dispositif pour la mise en oeuvre du procédé selon la revendication 1, caractérisé par un tambour de mesure (3) portant le produit plat et sur lequel on amène le produit plat (1) et par un moyen capteur optique (6 à 12) disposé le long d'une tangente au tambour et qui comprend une source lumineuse (6) disposée d'un côté du tambour et une ligne de diodes (12), disposée de l'autre côté du tambour et qui est orientée perpendiculairement au plan tangentiel.

8.  Dispositif selon la revendication 7, caractérisé par le fait que le moyen capteur (6 à 12) est déplaçable dans un sens et dans l'autre le long du tambour (3).

9.  Dispositif selon la revendication 7, caractérisé par le fait que d'un côté du tambour sont disposées plusieurs sources lumineuses (6) auxquelles sont associées, de l'autre côté du tambour, soit un miroir pour renvoyer les rayons lumineux tous sur la même ligne de diodes (12), soit des lignes de diodes individuelles respectives.

10. Dispositif pour la mise en oeuvre du procédé selon la revendication 1, caractérisé par le fait que la surface déflectrice (2) est formée par un tambour de mesure (3) tournant ou par une pièce en forme de glaive fixe (20) et qu'à une des extrémités longitudinales du tambour (3) ou du glaive (20) est disposée une source lumineuse (6) ou un émetteur laser et, à l'autre extrémité est disposé un récepteur optique (12), par exemple une diode de ligne, l'émetteur et le récepteur étant réglables en hauteur de façon synchrone.

Fig.1

Fig.2

≈ 0,5mm

Fig.3

Fig.4

Fig.5